# EUROPÄISCHE PATENTSCHRIFT

(11) **EP 2 125 696 B1**
(45) Veröffentlichungstag und Bekanntmachung des Hinweises auf die Patenterteilung: **31.10.2012**
(21) Anmeldenummer: 07857794.7
(22) Anmeldetag: 19.12.2007
(51) Int. Cl.: C07C 209/48, C07C 209/52

(54) **KONTINUIERLICHES VERFAHREN ZUR HYDRIERUNG VON 3-CYANO-3,5,5-TRIMETHYL-CYCLOHEXYLIMIN**
CONTINUOUS PROCESS FOR THE HYDROGENATION OF 3-CYANO-3,5,5-TRIMETHYL-CYCLOHEXYLIMINE
PROCÉDÉ CONTINU D'HYDROGÉNATION DE 3-CYANO-3,5,5-TRIMÉTHYL-CYCLOHEXYLIMINE

(30) Priorität: 22.12.2006 EP 06127089
(43) Veröffentlichungstag der Anmeldung: 02.12.2009
(73) Patentinhaber: BASF SE, 67056 Ludwigshafen (DE)
(72) Erfinder: ERNST, Martin, 69115 Heidelberg (DE); HILL, Thomas, 67071 Ludwigshafen (DE); MAKARCZYK, Piotr, 67063 Ludwigshafen (DE); MELDER, Johann-Peter, 67459 Böhl-Iggelheim (DE)
(86) Internationale Anmeldenummer: PCT/EP2007/064171
(87) Internationale Veröffentlichungsnummer: WO 2008/077852

(56) Entgegenhaltungen:
- EP-A- 0 449 089
- DE-C1- 19 507 398
- DE-C1- 19 747 913

## Beschreibung

Die vorliegende Erfindung betrifft ein kontinuierliches Verfahren zur Herstellung von 3-Aminomethyl-3,5,5-trimethyl-cyclohexylamin durch Umsetzung eines Eduktstroms enthaltend 3-Cyano-3,5,5-trimethyl-cyclohexylimin mit Wasserstoff und Ammoniak an Hydrierkatalysatoren.

3-Aminomethyl-3,5,5-trimethyl-cyclohexylamin (Isophorondiamin, IPDA) ist ein wichtiges Zwischenprodukt für Polyamide und Epoxidharze und zur Herstellung des Folgeproduktes Isophorondiisocyanat (IPDI), das als Komponente in Polyurethanen Verwendung findet.

Die Herstellung von 3-Aminomethyl-3,5,5-trimethyl-cyclohexylamin erfolgt im industriellen Maßstab durch Umsetzung von 3-Cyano-3,5,5-trimethyl-cyclohexanon (Isophoronnitril, IPN) mit Ammoniak zu 3-Cyano-3,5,5-trimethyl-cyclohexylimin (Isophoronnitrilimin, IPNI).
In einer reduktiven Aminierungsreaktion wird IPNI anschließend katalytisch mit Wasserstoff in Gegenwart von Ammoniak zu 3-Cyano-3,5,5-trimethyl-cyclohexylamin (Isophorondiamin, IPDA) umgesetzt.

Zur Steigerung der Ausbeute kann die reduktive Aminierung in mehreren Stufen erfolgen. So beschreibt EP-A1-0394968 ein mehrstufiges Verfahren, in dem zunächst selektiv die Iminogruppe von IPNI hydriert wird und anschließend unter drastischeren Reaktionsbedingungen (höherer Druck und Temperatur) die Hydrierung der Nitrilgruppe erfolgt. Offenbarungsgemäß kann durch eine solche Reaktionsführung die Bildung von 3-Cyano-3,5,5-trimethyl-cyclohexanol, welches durch Reduktion des mit IPNI im Gleichgewicht stehenden 3-Cyano-3,5,5-trimethyl-cyclohexanons entsteht, verringert werden. Der Anteil an weiteren Nebenprodukten, wie Zyklen, beträgt in den Beispielen jedoch zwischen 3 und 7%.

Gute Ausbeuten werden erzielt, wenn die reduktive Aminierung in Gegenwart von basischen Katalysatoren oder Verbindungen durchgeführt wird. So wird in DE-A-4010227 die reduktive Aminierung zum Teil in Gegenwart von basischen Katalysatoren durchgeführt, wobei gute Ausbeuten erzielt werden.

In EP-A1-0623585 wird gezeigt, dass eine Dotierung von Katalysatoren mit basischen Komponenten zu höheren Ausbeuten bei der reduktiven Aminierung führt.

DE-C-19747913 beschreibt ein Verfahren zur Hydrierung von Iminen und Nitrilen zu Aminen, insbesondere IPDA, wobei die Ausbeute durch Zugabe eines quaternären Ammoniumhydroxids gesteigert wird.

Bei der Herstellung von IPDA ist neben der Erzielung einer hohen Produktausbeute die Steuerung des Isomerenverhältnisses zwischen cis-Isophorondiamin und trans-Isophorondiamin bei der reduktiven Aminierung von hoher Bedeutung, da sich die Isomere hinsichtlich ihrer Reaktivitäten unterscheiden. Somit wird durch das cis-trans-Verhältnis die Weiterverarbeitung von IPDA und dessen Folgeprodukt IPDI und damit auch die Produkteigenschaften der aus diesen Rohstoffen hergestellten Erzeugnisse, beeinflusst. Marktübliches IPDI weist in der Regel ein cis-trans-Verhältnis (CTV) von 74:26 bis 78:22 auf.

Ein solches Isomerenverhältnis kann durch den in EP-A1-0394968 beschriebenen Prozess eingestellt werden. In einer ersten Reaktionsstufe erfolgt die Hydrierung von Isophoronnitril (IPN) in einem Temperaturbereich von 10 bis 90°C und in der anschließenden zweiten Reaktionsstufe bei 90 bis 160°C, wobei die Temperaturunterschied zwischen der ersten und der zweiten Reaktionsstufe mindestens 30°C beträgt und die Verweilzeit in der ersten Reaktionsstufe kürzer ist als in der zweiten Reaktionsstufe: Durch die Variation der Temperatur der ersten Reaktionsstufe konnte ein CTV von 55:45 bis 80:20 eingestellt werden, wobei die Ausbeute bei einem CTV von 76:24 ein Maximum durchlief.

Gemäß DE-C-19507398 hat auch die in der hydrierenden Aminierung eingesetzte Basenmenge einen Einfluss auf das Isomerenverhältnis. So konnte eine Erhöhung des CTV von 60:40 auf 68:32 durch Erniedrigung der Basenkonzentration erzielt werden. Die Verringerung der Basenkonzentration führte jedoch auch zu einer Absenkung der Ausbeute von 97 auf ca. 92%.

Eine weitere Steigerung des CTV auf 75:25 konnte in DE-A-19756400 bei der Durchführung der reduktiven Aminierung in Gegenwart einer Säure erreicht werden. Die Ausbeuten lagen im Bereich von 92%.

Auch der in der reduktiven Aminierung verwendete Katalysator kann das Isomerenverhältnis beeinflussen. So wird in DE-A-4343890 berichtet, dass das CTV durch die Verwendung von Ruthenium gegenüber Kobalt- oder kobalthaltigen Katalysatoren erhöht wird, die Ausbeute jedoch abnimmt.

Mittels dieser Erfindung wird ein Verfahren zur Verfügung gestellt, das es ermöglicht das Isomerenverhältnis bei zumindest gleichbleibender oder verbesserter Ausbeute zu erhöhen und hohe Raum-Zeit-Ausbeuten zu erzielen. Insbesondere soll mittels dieses Verfahrens die Bildung von Nebenprodukten verhindert werden, die schwer aus dem Reaktionsgemisch abzutrennen sind. Weiterhin soll die Verfahrenökonomie durch eine verbesserte Ausbeute gesteigert werden.

Demgemäss wurde ein kontinuierliches Verfahren zur Herstellung von 3-Aminomethyl-3,5,5-trimethyl-cyclohexylamin durch Umsetzung eines Eduktstroms enthaltend 3-Cyano-3,5,5-trimethyl-cyclohexylimin mit Wasserstoff und Ammoniak an Hydrierkatalysatoren gefunden, welches dadurch gekennzeichnet ist, dass man die Basizität des Reaktionsgemisches während der Umsetzung erhöht, in dem man das Reaktionsgemisch mit einer basischen Verbindung ungleich Ammoniak und/oder einem basischen Katalysator in Kontakt bringt, nachdem ein Teil des 3-Cyano-3,5,5-trimethyl-cyclohexylimins umgesetzt wurde.

Den 3-Cyano-3,5,5-trimethyl-cyclohexylimin-haltigen Eduktstrom erhält man in der Regel durch Umsetzung von 3-Cyano-3,5,5-trimethyl-cyclohexanon (IPN) mit überschüssigen Ammoniak in Gegenwart eines Iminbildungskatalysators (Iminierung).

Als Iminbildungskatalysator kommen beispielsweise feste Brönstedt- oder LewisSäuren in Betracht, wie sie beispielsweise in der EP-A1-449089 (Seite 2, Spalte 2, Zeilen 11-20) und in dem Artikel von Tanabe et al. (K. Tanabe, Studies in Surface Science and Catalysis, Vol. 51, 1989,. S. 1 ff) beschrieben sind. Beispielhaft seien hier acide Metalloxidkatalysatoren, wie Aluminiumoxid, Titandioxid, Zirkoniumdioxid und Siliciumdioxid genannt. Weiterhin kommen mit Ammonium-Ionen beladene anorganische oder organische Ionenaustauscher, wie Zeolithe oder sulfonierte Copolymerisate aus Styrol und Divenylbenzol (z.B. der Marken Lewatit® der Fa. Lanxess, Amberlite® der Fa. Rohm & Haas) oder Ionenaustauscher auf Basis Siloxan (z.B. der Marke Deloxan® der Fa. Degussa) in Betracht.

Pro mol eingesetztes IPN werden üblicherweise 5 bis 500 Mol Ammoniak (NH₃), bevorzugt 10 bis 400 mol NH₃, besonders bevorzugt 20 bis 300 mol NH₃, eingesetzt.

Die Iminierung von IPN kann in Anwesenheit eines Lösungsmittels erfolgen, z.B. in Alkanolen oder Ethern, wie Ethanol, Butanol oder Tetrahydrofuran (THF). Bevorzugt wird die Iminierung von IPN ohne eines Zusatzes von Lösungsmittel durchgeführt.

Die Iminierung wird bevorzugt kontinuierlich durchgeführt, üblicherweise in Druckbehältern oder Druckbehälterkaskaden. Bevorzugt werden IPN und NH₃ durch einen Rohrreaktor geleitet, in dem der Iminibildungskatalysator in Form eines Festbetts angeordnet ist.

Die Iminierung wird bevorzugt in einem Temperaturbereich von 20 bis 150°C, vorzugsweise 30 bis 130°C und besonders bevorzugt bei 50 bis 100°C durchgeführt.

Der Druck bei der Iminierung beträgt in der Regel von 50 bis 300 bar, bevorzugt 100 bis 250 bar.

In der Regel stellt man bei der Iminierung eine Katalysatorbelastung von 0,01 bis 10, vorzugsweise von 0,05 bis 7, besonders bevorzugt von 0,1 bis 5 kg IPN pro kg Katalysator und Stunde ein.

Der Reaktionsaustrag aus der Iminierung enthält üblicherweise IPNI und Ammoniak und nicht umgesetztes IPN. Der Umsatz von IPN zu IPNI liegt üblicherweise bei mehr als 80%, vorzugsweise mehr als 90% und besonders bevorzugt mehr als 95%.

Der Reaktionsaustrag aus der Iminierung wird als 3-Cyano-3,5,5-trimethyl-cyclohexylimin-haltiger Eduktstrom mit Wasserstoff und Ammoniak an Hydrierkatalysatoren umgesetzt (reduktive Aminierung).

Die Umsetzung des Eduktstroms enthaltend 3-Cyano-3,5,5-trimethyl-cyclohexylimin erfolgt bevorzugt in flüssigem Ammoniak. Pro mol IPNI (3-Cyano-3,5,5-trimethylcyclohexylimin) setzt man üblicherweise 5 bis 500 mol NH₃, bevorzugt 10 bis 400 mol NH₃ und besonders bevorzugt 20 bis 300 mol NH₃ ein. Zweckmäßigerweise stellt man bei der vorgelagerten Iminierung das Molverhältnis zwischen IPN und NH₃ so ein, dass das Molverhältnis auch bei der reduktiven Aminierung in einem geeigneten Bereich liegt. Der NH₃-Anteil kann jedoch vor der reduktiven Aminierung durch Zugabe von zusätzlichem NH₃ auf einen gewünschten Wert erhöht werden.

Als weiterer Ausgangsstoff für die Umsetzung des 3-Cyano-3,5,5-trimethyl-cyclohexylimin-haltiger Eduktstrom wird Wasserstoff eingesetzt. Das Molverhältnis zwischen Wasserstoff und IPNI beträgt in der Regel 3 bis 10 000 zu 1, bevorzugt von 4 bis 5000 zu 1 und besonders bevorzugt von 5 bis 1000 zu 1.

Der Wasserstoff wird dem 3-Cyano-3,5,5-trimethyl-cyclohexylimin-haltigen Eduktstrom bevorzugt nach der Iminierung und vor der reduktiven Aminierung zugeführt. Es ist jedoch auch denkbar, dass der Wasserstoff bereits vor der Iminierung zugeführt wird, da die Iminierung üblicherweise an Katalysatoren erfolgt, die die Hydrierung nicht katalysieren. Somit kann auch vor der Iminierung zugeführter Wasserstoff als Ausgangsstoff für die Umsetzung des 3-Cyano-3,5,5-trimethyl-cyclohexylimin-haltiger Eduktstroms während der reduktiven Aminierung zur Verfügung stehen.

Als Hydrierkatalysatoren können prinzipiell alle Hydrierkatalysatoren eingesetzt werden, die Nickel, Kobalt, Eisen, Kupfer, Ruthenium und/oder andere Metalle der VIII. Nebengruppe des Periodensystems enthalten. Als Hydrierkatalysatoren sind weiterhin Katalysatoren geeignet, die die Elemente Chrom, Mangan Kupfer, Molybdän, Wolfram und/oder Rhenium enthalten.

Bevorzugt verwendet man Hydrierkatalysatoren, die Ruthenium, Kobalt und/oder Nickel enthalten. Besonders bevorzugt sind Katalysatoren die Ruthenium und/oder Kobalt enthalten.
Die oben genannten Hydrierkatalysatoren können in üblicher Weise mit Promotoren, beispielsweise mit Chrom, Eisen, Kobalt, Mangan, Thallium, Molybdän, Titan und/oder Phosphor dotiert werden.

Die katalytisch aktiven Metalle können als Vollkontakte oder auf Trägern eingesetzt werden. Als solche Träger kommen z.B. Alumumiumoxid, Titandioxid, Zirkoniumdioxid oder Magnesiumoxid/Aluminiumoxid in Betracht. Die Träger können auch iminieraktiv sein, um die Umsetzung von während der Hydrierung der Imingruppe im Gleichgewicht mit dem Imin vorliegendem Keton zu ermöglichen.

Die katalytisch aktiven Metalle können auch in Form von Schwammkatalysatoren, sogenannten Raney-Katalysatoren, eingesetzt werden. Als Raney-Katalysatoren werden bevorzugt Raney-Kobalt-Katalysatoren, Raney-Nickel-Katalysatoren und/oder Raney-Kupfer-Katalysatoren eingesetzt. Besonders bevorzugt werden Raney-Kobalt-Katalysatoren verwendet.

Als Hydrierkatalysatoren können auch vorteilhaft selektive Hydrierkatalysatoren eingesetzt werden, wobei unter selektiven Hydrierkatalysatoren solche Katalysatoren zu verstehen sind, die bevorzugt die Imingruppe gegenüber der Nitrilgruppe des 3-Cyano-3,5,5-trimethyl-cyclohexylimins hydrieren.

Selektive Hydrierkatalysatoren sind beispielsweise Hydrierkatalysatoren, die Ruthenium, Palladium und/oder Rhodium enthalten. Bevorzugte selektive Hydrierkatalysatoren enthalten Ruthenium und/oder Rhodium und besonders bevorzugte Hydrierkatalysatoren enthalten Ruthenium.

Die reduktive Aminierung wird vorzugsweise kontinuierlich in Druckbehältern durchgeführt. Insbesondere ist für diese Reaktion ein Rohrreaktor mit Katalysatorfestbett geeignet.

Die Katalysatorbelastung bei kontinuierlicher Fahrweise liegt typischerweise bei 0,01 bis 10, vorzugsweise von 0,05 bis 7, besonders bevorzugt von 0,1 bis 5 kg IPNI pro kg Katalysator und Stunde.

Erfindungsgemäß erhöht man die Basizität des Reaktionsgemisches während der Umsetzung, in dem man das Reaktionsgemisch mit einer basischen Verbindung ungleich Ammoniak und/oder einem basischen Katalysator in Kontakt bringt, nachdem ein Teil des 3-Cyano-3,5,5-trimethyl-cyclohexylimins umgesetzt wurde.

Die Basizität des Reaktionsgemisches enthaltend 3-Cyano-3,5,5-trimethyl-cyclohexylimin, Ammoniak, Wasserstoff und den Hydrierkatalysator kann dadurch erhöht werden, dass man das Reaktionsgemisch mit einer basischen Verbindung in Kontakt bringt.

Dabei versteht sich, dass der Begriff basische Verbindung nicht das Edukt Ammoniak erfasst, sondern eine oder mehrere der untenstehend aufgeführten Verbindungen umfasst oder solche Verbindungen, die in analoger Weise wie die untenstehend aufgeführten Verbindungen wirken.

So kann die Basizität des Reaktionsgemisches dadurch erhöht werden, dass man dem Reaktionsgemisch eine basische Verbindung zufügt.
In einer weiteren Ausführungsform kann die Basizität des Reaktionsgemischs dadurch erhöht werden, dass man einen basischen Hydrierkatalysator mit dem Reaktionsgemisch in Kontakt bringt.

Als geeignete basische Verbindungen kommen basische Metallverbindungen, wie die Oxide, Hydroxide oder Carbonate der Alkali-, Erdalkalkali- oder Seltenerdmetalle in Betracht.

Bevorzugt sind die Metallverbindungen der Alkali- und Erdalkalimetalle, wie die entsprechenden Oxide, Hydroxide und Carbonate, wie Li₂O, Na₂O, K₂O, Rb₂O, Cs₂O, Li-OH, NaOH, KOH, RbOH, CsOH, Li₂CO₃, Na₂CO₃, K₂CO₃, Cs₂CO₃, Rb₂CO₃, MgO, CaO, SrO, BaO, Mg(OH)₂, Ca(OH)₂, Sr(OH)₂, Ba(OH)₂, MgCO₃, CaCO₃, SrCO₃ oder BaCO₃. Besonders bevorzugt sind LiOH, NaOH oder KOH.

Ebenfalls geeignete, bevorzugte basische Verbindungen sind Amine oder Ammoniumhydroxide.

Besonders bevorzugt werden Lösungen der basischen Verbindungen in Wasser oder anderen geeigneten Lösungsmitteln, wie Alkanolen, wie C₁-C₄-Alkanolen, z.B. Methanol oder Ethanol, oder Ether, wie cyclische Ether, z.B. THF oder Dioxan, dem Reaktionsgemisch zugefügt. Besonders bevorzugt werden Lösungen von Alkali- oder Erdalkalihydroxiden in Wasser, besonders bevorzugt Lösungen von LiOH, NaOH oder KOH in Wasser, zugegeben.

Bevorzugt beträgt die Konzentration der basischen Verbindung in Wasser oder anderen geeigneten Lösungsmitteln 0,01 bis 20 Gew.-%, bevorzugt 0,1 bis 10 und besonders bevorzugt 0,2 bis 5 Gew.-%.

Die Menge der zugefügten Lösung der basischen Verbindung wird üblicherweise so gewählt, dass das Verhältnis der Masse der zugefügten basischen Verbindung zur Masse des 3-Cyano-3,5,5-trimethyl-cyclohexylimin im Eduktstrom 100 bis 10 000 zu 1 000 000 beträgt, bevorzugt 150 bis 5000 zu 1 000 000 und besonders bevorzugt 200 bis 1000 zu 1 000 000.

Im Rahmen dieser Erfindung kann die Basizität auch dadurch erhöht werden, dass man basische Hydrierkatalysatoren einsetzt. Solche basischen Hydrierkatalysatoren sind obengenannte Hydrierkatalysatoren, welche mit basischen Komponenten, wie Oxiden oder Hydroxiden von Alkali-, Erdalkali- und Seltenerdmetallen, dotiert und/oder auf basischen Trägern aufgebracht wurden.

Geeignete basische Träger für Hydrierkatalysatoren sind beispielsweise β-Aluminiumoxid oder Magnesiumoxid/Aluminiumoxid-Gemische, wobei der Anteil des Magnesiumoxids vorzugsweise 5 bis 40 Gew.-% beträgt. Dabei kann der Magnesiumoxid und Aluminiumoxid enthaltende Träger amorph sein oder als Spinell vorliegen. Katalysatoren auf basischen Trägern erhält man technisch in an sich bekannter Weise. So gewinnt man z.B. Ruthenium auf basischen Träger durch Auftragen von wässrigen Rutheniumsalz-Lösungen, wie Rutheniumchlorid und Rutheniumnitrat auf den entsprechenden basischen Träger.

Die Konzentration der Metalle, insbesondere Ruthenium, auf den basischen Trägern beträgt in der Regel 0,1 bis 10 Gew.-%, bevorzugt 0,5 bis 5 Gew.-% und besonders bevorzugt 1 bis 4 Gew.-%.

Unter basischen Katalysatoren versteht man auch solche Hydrierkatalysatoren, die mit den oben genannten basischen Komponenten, wie Oxiden oder Hydroxiden von Alkali-, Erdalkali- und Seltenerdmetallen, dotiert werden. Bevorzugt enthalten basische Katalysatoren mindestens eine basische Komponente, wie Li₂O, Na₂O, K₂O, MgO, CaO, SrO oder BaO.

Der Anteil an basischen Komponenten, d.h. basischen Dotierungen in basischen Hydrierkatalysatoren beträgt in der Regel mehr als 0,5 Gew.-% und besonders bevorzugt mehr als 0,7 Gew.-% und besonders bevorzugt mehr als 1 Gew.-% bezogen auf die Gesamtmasse des basischen Hydrierkatalysators.

Die eingangs beschriebenen Hydrierkatalysatoren, die nicht wie oben beschrieben auf basischen Trägern aufgebracht wurden und/oder die 0,5 Gew.-% oder weniger basische Komponenten, d.h. basischen Dotierungen, bezogen auf die Gesamtmasse des Katalysators enthalten, werden im folgenden als nicht-basische Hydrierkatalysatoren bezeichnet.

Erfindungsgemäß wird die Basizität des Reaktionsgemisches während der Umsetzung erhöht, in dem man das Reaktionsgemisch mit einer basischen Verbindung in Kontakt bringt, nachdem ein Teil des 3-Cyano-3,5,5-trimethyl-cyclohexylimins umgesetzt wurde.

Dabei erfolgt in der Regel die Erhöhung der Basizität durch Inkontaktbringen des Reaktionsgemischs mit der basischen Verbindung, nachdem 1 bis 95%, bevorzugt 5 bis 80% und besonders bevorzugt 10 bis 40% des 3-Cyano-3,5,5-trimethyl-cyclohexylimin im Eduktstrom umgesetzt wurden.

Vor Erhöhung der Basizität werden dem Reaktionsgemisch in der Regel keine basischen Verbindungen zugegeben. Es ist jedoch möglich, dass das Reaktionsgemisch geringe Mengen basischer Verbindungen enthält. Vorzugsweise beträgt das Verhältnis der Masse der basischen Verbindung zur Masse des 3-Cyano-3,5,5-trimethylcyclohexylimins im Eduktstrom vor der Erhöhung der Basizität jedoch weniger als 100 zu 1 000 000, vorzugsweise weniger als 50 zu 1 000 000.

Vor der Erhöhung der Basizität wird das Reaktionsgemisch üblicherweise mit nicht-basischen Katalysatoren in Kontakt gebracht.

Die reduktive Aminierung, d.h. die Umsetzung des 3-Cyano-3,5,5-trimethyl-cyclohexylimin-haltigen Eduktstroms mit Wasserstoff und Ammoniak an Hydrierkatalysatoren kann in einem oder in mehreren voneinander getrennten Reaktionsräumen erfolgen.

Wird die reduktive Amierung in nur einem Reaktionsraum durchgeführt, beispielsweise in einem Festbettreaktor, so kann die Erhöhung der Basizität durch das Inkontaktbringen des Reaktionsgemischs mit der basischen Verbindung in der Weise erfolgen, dass die Dosierung der basischen Verbindung zwischen dem Reaktoreingang, in den der 3-Cyano-3,5,5-trimethyl-cyclohexylimin-haltige Eduktstrom zusammen mit Ammoniak und Wasserstoff zugeführt wird, und dem Reaktorausgang erfolgt. Das Inkontaktbringen des Eduktstroms mit der basischen Verbindung kann erfindungsgemäß nicht vor der reduktiven Aminierung erfolgen.

Da wie oben beschrieben, die Reaktion bevorzugt unter einem hohen Druck erfolgt, ist es deshalb erforderlich eine Dosierung der basischen Verbindung bei einem hohen Betriebsdruck in dem Reaktor vorzunehmen. Geeignete technische Vorrichtung zur Dosierung von Stoffen unter Hochdruckbedingungen sind dem Fachmann bekannt. Insbesondere können Pumpen, wie Hochdruckpumpen bzw. Kolbenpumpen zur Dosierung von Stoffen unter Hochdruckbedingungen verwendet werden.

Es ist jedoch auch möglich, dass die Erhöhung der Basizität des Reaktionsgemisch durch das Inkontaktbringen mit einem basischen Katalysator in der Art erfolgt, dass zunächst der 3-Cyano-3,5,5-trimethyl-cyclohexylimin-haltiger Eduktstrom mit Wasserstoff und Ammoniak über einen der eingangs beschriebenen nicht-basischen Hydrierkatalysatoren und nachfolgend über einen basischen Hydrierkatalysator geleitet wird. Dies kann dadurch realisiert werden, dass die Katalysatoren in geeigneter Weise geschichtet sind.

Vorteilhafter Weise wird am Übergang zwischen der Schicht des nicht-basischen Hydrierkatalysators und des basischen Hydrierkatalysators, wie oben beschrieben, eine basische Verbindung zudosiert, da die basischen Komponenten des Hydrierkatalysators mit zunehmender Betriebsdauer ausgewaschen werden können.

Die reduktive Aminierung erfolgt bei Temperaturen von 50 bis 160°C und einem Druck von 50 bis 300 bar.

Üblicherweise ist das Temperaturprofil zwischen Reaktoreingang und Reaktorausgang weitestgehend konstant und durch die bei der reduktiven Aminierung freiwerdenden Reaktionswärme bestimmt.
Es ist jedoch auch möglich, ein Temperaturprofil zwischen Reaktoreingang und Reaktorausgang einzustellen. Die Ausbildung eines solchen Temperaturprofils kann dadurch realisiert werden, dass einzelne Bereiche des Reaktors getrennt und individuell voneinander einstellbar temperiert werden können. In einem solchen Falle ist es vorteilhaft, wenn die Temperatur zwischen Reaktoreingang und Reaktorausgang erhöht wird. Vorzugsweise beträgt die Temperatur am Reaktoreingang im Bereich von 50 bis 100°C, während die Temperatur am Reaktorausgang zwischen 100 und 160°C liegt. Das zunehmende Temperaturprofil zwischen Reaktoreingang und Reaktorausgang kann eine stetige Funktion sein oder in diskreten Schritten abnehmen.

In einer bevorzugten Ausführungsform erfolgt die reduktive Aminierung jedoch in zwei oder mehreren Stufen, wobei die Stufen in getrennten Reaktionsräumen erfolgen.

In einer besonders bevorzugten Ausführungsform wird die reduktive Aminierung in zwei Stufen durchgeführt, wobei die Stufen in getrennten Reaktionsräumen erfolgen.

Die erste Stufe (Stufe I) wird in der Regel in einem Temperaturbereich 50 bis 100°C, bevorzugt bei 55 bis 95°C und besonders bevorzugt bei 60 bis 90°C und bei einem Druck von 15 bis 300, vorzugsweise 20 bis 250 und besonders bevorzugt bei 30 bis 230 bar durchgeführt.

Die zweite Stufe (Stufe II) wird üblicherweise in einem Temperaturbereich von 70 bis 160°C, bevorzugt 75 bis 150°C und besonders bevorzugt bei 80 bis 140°C und bei einem Druck von 50 bis 300, vorzugsweise 80 bis 250 und besonders bevorzugt bei 100 bis 230 bar durchgeführt.

Beide Stufen werden üblicherweise jeweils in Druckbehältern, insbesondere in Festbettreaktoren durchgeführt.

Als Katalysatoren können in beide Stufen die eingangs beschriebenen nicht-basischen Hydrierkatalysatoren eingesetzt werden, wobei bevorzugt ein nicht-basischer Katalysator eingesetzt wird, der Kobalt enthält.

In einer bevorzugten Ausführungsform werden in die Stufe I als nicht-basische Hydrierkatalysatoren die eingangs beschriebenen selektiven Hydrierkatalysatoren eingesetzt.

Die Erhöhung der Basizität des Reaktionsgemisches durch Inkontaktbringen des Reaktionsgemischs mit der basischen Verbindung erfolgt in den oben beschriebenen Ausführungsformen bevorzugt, indem zwischen dem Ausgang der Stufe I und dem Eingang der Stufe II eine Lösung einer basischen Verbindung zudosiert wird.

Die Erhöhung der Basizität des Reaktionsgemisches durch das Inkontaktbringen mit einer basischen Verbindung kann aber auch in der Art erfolgen, dass in der Stufe I einer der eingangs beschriebenen nicht-basischen Hydrierkatalysatoren und in die Stufe II ein basischer Hydrierkatalysator eingesetzt wird.
Da die basischen Komponenten aus dem basischen Katalysator mit zunehmender Betriebsdauer ausgewaschen werden können, ist es vorteilhaft, wenn zwischen dem Ausgang der Stufe I und dem Eingang der Stufe II zusätzliche eine Lösung einer basischen Verbindung zudosiert wird.

In weiteren Ausführungsformen der Erfindung ist es möglich, sowohl die Stufe I als auch die Stufe II in weitere Teilstufen zu unterteilen, wobei auch die Teilstufen in jeweils getrennten Reaktionsräumen ausgeführt werden.

So ist es möglich, die Teilstufen der Stufe I in zwei oder mehreren Druckbehältern, insbesondere Festbettreaktoren, auszuführen. -

Wie oben beschrieben werden die Teilstufen der Stufe I üblicherweise in einem Temperaturbereich von 50 bis 100°C und bei einem Druck von 15 bis 300 bar durchgeführt. Druck und Temperatur können in den Teilstufen gleich oder verschieden voneinander sein. Vorteilhafter Weise werden die Teilstufen bei gleicher Temperatur und gleichem Druck betrieben. Wenn die Teilstufen bei unterschiedlichen Temperaturen und Drücken betrieben werden ist es vorteilhaft, wenn Druck und Temperatur von Teilstufe zu Teilstufe zunehmen, d.h. dass der Druck und die Temperatur in der ersten Teilstufe am niedrigsten sein sollten.

In jeder Teilstufe können die eingangs beschriebenen nicht-basischen Hydrierkatalysatoren eingesetzt werden. ,

In einer bevorzugten Ausführungsform werden in der ersten Teilstufe oder in den ersten Teilstufen der ersten Reaktionsstufe als nicht-basische Hydrierkatalysatoren selektive Hydrierkatalysatoren eingesetzt.

Aus Gründen der Verfahrensökonomie ist es vorteilhaft, wenn die Stufe I der reduktiven Aminierung aus nicht mehr als drei, vorzugsweise zwei und besonders bevorzugt einer Teilstufe besteht, da das Investment mit zunehmender Anzahl von Reaktoren zunimmt.

Wird die Stufe I der reduktiven Aminierung in nur einer Teilstufe durchgeführt, so ist es vorteilhaft, wenn die Basizität des Reaktionsgemisches erhöht wird, in dem die basische Verbindung mit dem Reaktionsgemisch nach dem Ausgang der Stufe I in Kontakt gebracht wird

Wird die Stufe I der reduktiven Aminierung in zwei oder mehr Teilstufen durchgeführt, so ist es empfehlenswert die Basizität des Reaktionsgemisches zu erhöhen, indem das Inkontaktbringen des Reaktionsgemischs mit der basischen Verbindung nach der ersten Teilstufe der Stufe I vorgenommen wird.

Vorzugsweise wird das Reaktionsgemisch mit der basischen Verbindung in Kontakt gebracht, in dem die basische Verbindung zwischen dem Ausgang einer Teilstufe und dem Eingang der darauffolgenden Teilstufe der Stufe I zudosiert wird.

Vorteilhafterweise erfolgt die Dosierung der basischen Verbindung zwischen der ersten Teilstufe und der zweiten Teilstufe der Stufe I. Es ist aber auch möglich die basische Verbindung zwischen dem Ausgang und dem Eingang von zwei beliebig aufeinanderfolgenden Teilstufen zu dosieren. Die Dosierung der basischen Verbindung darf aber erfindungsgemäß nicht vor der ersten Teilstufe der Stufe I erfolgen.

Die Erhöhung der Basizität des Reaktionsgemischs durch das Inkontaktbringen mit einem basischen Hydrierkatalysator kann auch in der Art erfolgen, dass in der ersten oder in den ersten Teilstufen einer der eingangs beschriebenen nicht-basischen Hydrierkatalysatoren eingesetzt wird und in einer der folgenden Teilstufen ein basischer Hydrierkatalysator verwendet wird. Es ist auch denkbar, dass in den Teilstufen eine Schichtung von nicht-basischen Hydrierkatalysatoren und basischen Hydrierkatalysatoren erfolgt.

Weiterhin ist es vorteilhaft in die Teilstufen mit basischen Hydrierkatalysatoren zusätzlich die Dosierung einer Lösung einer basischen Verbindung vorzunehmen, um die Möglichkeit des Auswaschens der basischen Komponenten des basischen Hydrierkatalysators zu kompensieren.

Ferner ist es möglich die Stufe II der reduktiven Aminierung in weitere Teilstufen zu unterteilen, wobei die Teilstufen vorzugsweise in jeweils getrennten Reaktionsräumen ausgeführt werden.

Die Teilstufen der Stufe II der reduktiven Aminierung werden wie oben beschrieben üblicherweise in einem Temperaturbereich von 70 bis 160°C und bei einem Druck von 50 bis 300 bar durchgeführt. Vorzugsweise werden die Teilstufen der Stufe II der reduktiven Aminierung in zwei oder mehreren Druckbehältern, insbesondere Festbettreaktoren, ausgeführt.

Vorzugsweise sollte die Erhöhung der Basizität des Reaktionsgemischs durch das Inkontaktbringen des Reaktionsgemischs mit einer basischen Verbindung und/oder einem basischen Hydrierkatalysator vor der Stufe II erfolgen. Es ist jedoch auch möglich, dass das Inkontaktbringen des Reaktionsgemischs in einer der Teilstufen der zweiten Reaktionsstufe vorzunehmen. Dies kann in analoger Weise dadurch erfolgen, dass eine Lösung einer basischen Verbindung zwischen den Teilstufen der Stufe II zudosiert wird, oder hinter der ersten Teilstufe der Stufe II ein basischer Hydrierkatalysator eingesetzt wird.
Weiterhin ist eine Schichtung von Hydrierkatalysatoren und basischen Hydrierkatalysatoren in den Teilstufen der Stufe II möglich.

Aus dem aus der reduktiven Aminierung erhaltenen Reaktionsaustrag werden NH₃ und Wasserstoff, gegebenenfalls unter Druck abgetrennt. Das so erhaltene Roh-IPDA lässt sich beispielsweise durch eine fraktionierende Rektifikation isolieren.

Es ist möglich das CTV im Reaktionsaustrag zu regulieren, indem der 3-Cyano-3,5,5-trimethyl-cyclohexylimin-haltige Eduktstrom vor Einleitung in die Stufe I der reduktiven Aminierung geteilt wird. Ein Teil wird zusammen mit Wasserstoff und NH₃ in die Stufe I oder in die erste Teilstufe der Stufe I geleitet, während der andere Teil in eine spätere Stufe (Stufe II) oder Teilstufe der Stufe I oder Stufe II zugeführt wird. Bevorzugt wird ein Teil des 3-Cyano-3,5,5-trimethyl-cyclohexylimin-haltigen Eduktstroms in die zweite Stufe der reduktiven Aminierung (Stufe II) zugeführt bzw. in eine Teilstufe der zweiten Stufe der reduktiven Aminierung.
In der Regel wird durch die Teilung des Eduktstromes das CTV abgesenkt, so dass durch die Regelung der Teilung des Eduktstromes das CTV eingestellt werden kann.

Eine weitere Steuerungsmöglichkeit des CTV besteht in der Regulierung der Temperatur in der ersten Teilstufe der Stufe I. In beiden Fällen wird letztlich der Umsatz des Eduktstroms in der ersten Teilstufe der Stufe I geregelt. Je höher der Umsatz in der Stufe I bzw. der ersten Teilstufe der Stufe I ist, desto höher ist das CTV im Produktstrom.

Mittels der beschriebenen Erfindung ist es möglich eine hohe IPDA-Ausbeute bei einem hohen CTV zu erzielen. Das dargestellte Verfahren kann mit einer hohen Raum-Zeit-Ausbeute betrieben werden. Die Bildung von störenden Nebenprodukten wird weitestgehend vermieden.

Die Erfindung wird in den nachfolgenden Beispielen erläutert.

### Beispiele:

### Vergleichsbeispiel 1

Es wurde eine Apparatur bestehend aus 5 Reaktoren verwendet.

In den ersten beiden Reaktoren wurde die Umsetzung von IPN zu IPNI (Iminierung) durchgeführt.
In den Reaktoren 3 bis 5 wurde der IPNI-haltige Reaktionsaustrag der Iminierung zu IPDA umgesetzt (reduktive Aminierung)

### Iminierung:

Die ersten beiden Reaktoren wurden mit γ-Aluminiumoxid (4mm Stränge) gefüllt.
Die Temperatur in dem ersten und zweiten Reaktor betrug jeweils 70°C.
In den ersten Reaktor wurden 14 g IPN pro Stunde und 62 g NH₃ pro Stunde zugeführt. Zusätzlich wurden 45 NI (Normliter) Wasserstoff pro Stunde bei einem Druck von 230 bar zugeführt.

### Reduktive Aminierung:

Der 3. und 4. Reaktor wurden mit einem nicht-basischen, selektiven Hydrierkatalysator (0,5 Gew.-% Ru auf einem γ-Aluminiumoxid-Träger (Degussa)) befüllt. Der 5. Reaktor wurde mit einem reduzierten Co-Katalysator (Zusammensetzung: Mn₃O₄: 5-6,2 Gew.% Na₂O: 0-0,5 Gew.-%, H₃PO₄: 2,8-3,8 Gew.-%, Rest Co + CoO) befüllt.
Die Temperatur im 3. Reaktor betrug 70°C (erste Teilstufe der Stufe I der reduktiven Aminierung). Die Temperatur im 4. Reaktor betrug 80°C (zweite Teilstufe der Stufe I der reduktiven Aminierung). Im 5. Reaktor betrug die Temperatur 120°C (Stufe II der reduktiven Aminierung).

Der IPNI-haltige Reaktionsaustrag aus der Iminierung wurde in den Eingang des 3. Reaktor (erste Teilstufe der Stufe I) eingeleitet.

Der Reaktionsaustrag nach 91 Stunden enthielt neben Ammoniak und Wasser laut gaschromatographischer Analyse 89,7% IPDA und 4,4% 1,3,3-Trimethyl-6-aza-bicyclo[3.2.1]octan (Bicyclus) sowie 3,2% 1,3,3-Trimethyl-6-aza-bicyclo[3.2.1]oct-7-ylideneamin (Amidin) neben 0,3% Aminonitril, entsprechend einer Selektivität von 90%. Das lsomerenverhältnis betrug 86/14 cisarans-IPDA.

### Beispiel 1

Das Beispiel wurde im analog zu Vergleichsbeispiel 1 durchgeführt.

Nach einer Laufzeit von insgesamt 91 Stunden wurde jedoch am Eingang des 5. Reaktor (Stufe II) 0,009 g pro Minute einer 1 Gew.-%-igen wässrigen Lösung von NaOH zudosiert. Alle anderen Parameter blieben gegenüber Beispiel 1 gleich. Der Reaktionsaustrag nach 146 Stunden enthielt neben Ammoniak und Wasser laut gaschromatographischer Analyse 93,3% IPDA, 1,5% Bicyclus und 2,6% Amidin neben 0,13 % Aminonitril, entsprechend einer Selektivität von 93,4%. Das Isomerenverhältnis betrug 85/15 cis:trans-IPDA.

Der Vergleich zwischen Vergleichsbeispiel 1 und Beispiel 1 zeigt, dass durch die Erhöhung der Basizität des Reaktionsgemisches zwischen Stufe I und Stufe II die Selektivität von 90 auf 93,4% erhöht werden konnte bei nahezu konstantem CTV.

### Vergleichsbeispiel 2

Es wurde eine Apparatur bestehend aus 5 Reaktoren verwendet.

In den ersten beiden Reaktoren wurde die Umsetzung von IPN zu IPNI (Iminierung) durchgeführt.
In den Reaktoren 3 bis 5 wurde der IPNI-haltige Reaktionsaustrag der Iminierung zu IPDA umgesetzt (reduktive Aminierung)

### Iminierung:

Die ersten beiden Reaktoren waren mit γ-Aluminiumoxid (4mm Stränge) gefüllt.
Die Temperatur in der Iminierung betrug 80°C.
Es wurden 54 g IPN pro Stunde und 239 g NH₃ pro Stunde in den ersten Reaktor zugeführt.
Zusätzlich wurden 81 NI Wasserstoff pro Stunde bei einem Druck von 230 bar zugeführt.

### Reduktive Aminierung:

Der 3., 4. und 5. Reaktor wurden mit einem nicht-basischen Hydrierkatalysator, nämlich einem reduzierten Co-Katalysator (Zusammensetzung: Mn₃O₄: 5-6,2 Gew.-% Na₂O: 0-0,5 Gew.-%, H₃PO₄: 2,8-3,8 Gew.-%, Rest Co + CoO) befüllt.
Die Temperatur im 3. Reaktor betrug 80°C (erste Teilstufe der Stufe I der reduktiven Aminierung), im 4. Reaktor 90°C (zweite Teilstufe der Stufe II der reduktiven Aminierung) und im 5. Reaktor 125°C (Stufe II der reduktiven Aminierung).

Der IPNI-haltige Reaktionsaustrag aus der Iminierung wurde in den 3. Reaktor eingeleitet (erste Teilstufe der Stufe I).

Der Reaktionsaustrag nach 820 Stunden enthielt neben Ammoniak und Wasser laut gaschromatographischer Analyse 94,2% IPDA und 0,8% 1,3,3-Trimethyl-6-aza-bicyclo[3.2.1]octan (Bicyclus) sowie 0,9% 1,3,3-Trimethyl-6-aza-bicyclo[3.2.1]oct-7-ylideneamin (Amidin) neben 0,8% Aminonitril, entsprechend einer Selektivität von 94,9%. Das Isomerenverhältnis betrug 72/28 cis:trans-IPDA.

### Beispiel 2

Das Beispiel 2 wurde in analoger Weise zu Vergleichsbeispiel 2 ausgeführt.

Nach 1000 h wurden jedoch zusätzlich 0,036 ml pro Minute einer 2 Gew.-%-igen Na-OH am Eingang des 4. Reaktor (zweite Teilstufe der Stufe I) zudosiert. Die Basendosierung erfolgte somit erfindungsgemäß zwischen der ersten und zweiten Teilstufe der Stufe I der reduktiven Aminierung. Alle anderen Parameter blieben gleich. Der Reaktionsaustrag nach 1275 Stunden enthielt neben Ammoniak und Wasser laut gaschromatographischer Analyse 95,8% IPDA und 0,5% 1,3,3-Trimethyl-6-aza-bicyclo[3.2.1]octan (Bicyclus) sowie 0,3% 1,3,3-Trimethyl-6-aza-bicyclo[3.2.1]oct-7-ylideneamin (Amidin) neben 0,6% Aminonitril, entsprechend einer Selektivität von 96,4%. Das Isomerenverhältnis betrug 72/28 cisarans-IPDA.

Der Vergleich zwischen Vergleichsbeispiel 2 und Beispiel 2 zeigt, dass durch die Erhöhung der Basizität des Reaktionsgemisches durch Zugabe einer NaOH-Lösung zwischen Stufe I und Stufe II die Selektivität von 94,9 auf 96,4% erhöht werden konnte bei konstantem CTV.

### Vergleichsbeispiel 2a

Dieser Versuch wurde analog zu Beispiel 2 ausgeführt, jedoch die Basendosierung vom Eingang des 4. Reaktors auf den Eingang des 3. Reaktors verlegt, d.h. die basische Verbindung wurde bereits vor der Stufe I der reduktiven Aminierung zugegeben. Alle anderen Parameter blieben gleich. Der Reaktionsaustrag nach 1300 Stunden enthielt neben Ammoniak und Wasser laut gaschromatographischer Analyse jetzt 95,3% IPDA und 0,7% 1,3,3-Trimethyl-6-aza-bicyclo[3.2.1]oct-7-ylideneamin (Amidin) neben 0,07% Aminonitril, entsprechend einer Selektivität von 95,4%. Das Isomerenverhältnis betrug 70/30 cis:trans-IPDA.

Wird die NaOH-Lösung zum Reaktionsgemisch bereits vor Beginn der Umsetzung des 3-Cyano-3,5,5-trimethyl-cyclohexylimin zugefügt, so erhält man im Vergleich zu Beispiel 2 verringerte Selektivitäten und Isomerenverhältnisse (CTV).

### Vergleichsbeispiel 3

Es wurde eine Apparatur bestehend aus 5 Reaktoren verwendet.

In den ersten beiden Reaktoren wurde die Umsetzung von IPN zu IPNI (Iminierung) durchgeführt.
In den Reaktoren 3 bis 5 wurde der IPNI-haltige Reaktionsaustrag der Iminierung zu IPDA umgesetzt (reduktive Aminierung)

### Iminierung:

Die ersten beiden Reaktoren wurden mit γ-Aluminiumoxid (4mm Stränge) gefüllt.
Die Temperatur in dem ersten und zweiten Reaktor betrug jeweils 70°C.
In den ersten Reaktor wurden 17 g IPN pro Stunde und 80 g NH₃ pro Stunde zugeführt. Zusätzlich wurden 20 NI Wasserstoff pro Stunde bei einem Druck von 230 bar zugeführt.

### Reduktive Aminierung:

Der 3. Reaktor wurden mit dem konventionellen, selektiven Hydrierkatalysator 2% Ru/Aluminiumoxid (Degussa) befüllt. Der 4. und 5. Reaktor wurde mit einem reduzierten Co-Katalysator (Zusammensetzung: Mn₃O₄: 5-6,2 Gew.-% Na₂O: 0-0,5 Gew.-%, H₃PO₄: 2,8-3,8 Gew.-%, Rest Co + CoO) befüllt. Die Temperatur im 3. Reaktor betrug 70°C (erste Teilstufe der Stufe I der reduktiven Aminierung). Die Temperatur im 4. Reaktor betrug 92°C (zweite Teilstufe der Stufe I der reduktiven Aminierung). Im 5. Reaktor betrug die Temperatur 135°C (Stufe II der reduktiven Aminierung).

70% des IPNI-haltigen Reaktionsaustrags aus der Iminierung wurden in den Eingang des 3. Reaktor (erste Teilstufe der Stufe I) eingeleitet.

Der Reaktionsaustrag nach 190 Stunden enthielt neben Ammoniak und Wasser laut gaschromatographischer Analyse 94,6% IPDA und 2,1% 1,3,3-Trimethyl-6-aza-bicyclo[3.2.1]octan (Bicyclus) sowie 0,7% 1,3,3-Trimethyl-6-aza-bicyclo[3.2.1]oct-7-ylideneamin (Amidin) neben 0,2% Aminonitril, entsprechend einer Selektivität von 95%. Das Isomerenverhältnis betrug 78/22 cisarans-IPDA.

### Beispiel 3

Das Beispiel wurde in analoger Weise zu Vergleichsbeispiel 1 durchgeführt.

Nach einer Laufzeit von insgesamt 269 Stunden wurde jedoch am Eingang des 4. Reaktors (Stufe II) 0,023 g pro Minute einer 0,25 Gew.-%-igen wässrigen Lösung von NaOH zudosiert. Alle anderen Parameter blieben gegenüber Beispiel 1 gleich. Der Reaktionsaustrag nach 509 Stunden enthielt neben Ammoniak und Wasser laut gaschromatographischer Analyse 97,0% IPDA, 0,87% Bicyclus und 0,25% Amidin neben 0,12% Aminonitril, entsprechend einer Selektivität von 97,1 %. Das Isomerenverhältnis betrug 75/25 cisarans-IPDA.

Durch Erhöhung der Temperatur nach 700h im 3. Reaktor auf 80°C konnte das cis:trans-verhältnis bei nahezu gleicher Selektivität (97,2%) auf 77/23 erhöht werden.

### Vergleichsbeispiel 4a

Es wurde eine Apparatur bestehend aus 4 Reaktoren verwendet.

In dem ersten Reaktor wurde die Umsetzung von IPN zu IPNI (Iminierung) durchgeführt.
In den Reaktoren 2 bis 4 wurde der IPNI-haltige Reaktionsaustrag der Iminierung zu IPDA umgesetzt (reduktive Aminierung)

### Iminierung:

Der erste Reaktor wurde mit Titandioxid (1,5 mm Stränge) gefüllt.
Die Temperatur in dem Reaktor betrug 80°C.

In den ersten Reaktor wurden 35 g IPN pro Stunde und 110 g NH₃ pro Stunde zugeführt. Zusätzlich wurden 84 NI Wasserstoff pro Stunde bei einem Druck von 230 bar zugeführt.

### Reduktive Aminierung:

Der 2., 3. und 4. Reaktor wurde mit einem reduzierten Co-Katalysator (Zusammensetzung: Mn₃O₄: 5 - 6,2 Gew.-% Na₂O: 0 - 0,5 Gew.-%, H₃PO₄: 2,8 - 3,8 Gew.-%, Rest Co + CoO) befüllt. Die Temperatur im 2. Reaktor betrug 70°C (erste Teilstufe der Stufe I der reduktiven Aminierung). Die Temperatur im 3. Reaktor betrug 90°C (zweite Teilstufe der Stufe I der reduktiven Aminierung). Im 4. Reaktor betrug die Temperatur 145°C (Stufe II der reduktiven Aminierung). Es wurde keine Base zudosiert.

Der Reaktionsaustrag nach 1198 Stunden enthielt neben Ammoniak und Wasser laut gaschromatographischer Analyse 97,0% IPDA und 0,9% 1,3,3-Trimethyl-6-aza-bicyclo[3.2.1]octan (Bicyclus) neben 0,3% Aminonitril, entsprechend einer Selektivität von 97,3%. Das Isomerenverhältnis betrug 71/29 cis:trans-IPDA.

### Vergleichsbeispiel 4b

Das Beispiel wurde in analoger Weise zu Vergleichsbeispiel 4a durchgeführt.

Nach einer Laufzeit von 1540 Stunden wurde jedoch am Eingang des 2. Reaktors (erste Teilstufe der Stufe I) 0,012 g pro Minute einer 1 Gew.-%-igen wässrigen Lösung von NaOH zudosiert. Alle anderen Parameter blieben gegenüber Vergleichsbeispiel 4a gleich. Der Reaktionsaustrag nach 1708 Stunden enthielt neben Ammoniak und Wasser laut gaschromatographischer Analyse 97,9% IPDA und 0,1% 1,3,3-Trimethyl-6-aza-bicyclo[3.2.1]octan (Bicyclus) neben 0,4% Aminonitril, entsprechend einer Selektivität von 98,4%. Das Isomerenverhältnis betrug 68/32 cis:trans-IPDA.

### Beispiel 4

Das Beispiel wurde in analoger Weise zu Vergleichsbeispiel 4b durchgeführt.

Nach einer Laufzeit von 1708 Stunden wurde die Basendosierung vom Eingang des 2. Reaktors (erste Teilstufe der Stufe I) auf den Eingang des 3. Reaktors gelegt (zweite Teilstufe der Stufe I). Alle anderen Parameter blieben gegenüber Vergleichsbeispiel 4b gleich. Der Reaktionsaustrag nach 1949 Stunden enthielt neben Ammoniak und Wasser laut gaschromatographischer Analyse 98,0% I PDA und 0, 1 % 1,3,3-Trimethyl-6-aza-bicyclo[3.2.1]octan (Bicyclus) neben 0,4% Aminonitril, entsprechend einer Selektivität von 98,4%. Das Isomerenverhältnis betrug 72/28 cis:trans-IPDA.

## Patentansprüche

1. Kontinuierliches Verfahren zur Herstellung von 3-Aminomethyl-3,5,5-trimethylcyclohexylamin durch Umsetzung eines Eduktstroms enthaltend 3-Cyano-3,5,5-trimethyl-cyclohexylimin mit Wasserstoff und Ammoniak an Hydrierkatalysatoren, **dadurch gekennzeichnet, dass** man die Basizität des Reaktionsgemisches während der Umsetzung erhöht, in dem man das Reaktionsgemisch mit einer basischen Verbindung ungleich Ammoniak und/oder einem basischen Katalysator in Kontakt bringt, nachdem ein Teil des 3-Cyano-3,5,5-trimethylcyclohexylimins umgesetzt wurde.

2. Verfahren nach Anspruch 1, **dadurch gekennzeichnet, dass** das Verhältnis der Masse der basischen Verbindung zur Masse des 3-Cyano-3,5,5-trimethylcyclohexylimins im Eduktstrom vor der Erhöhung der Basizität weniger als 100 zu 1 000 000 beträgt.

3. Verfahren nach mindestens einem der Ansprüche 1 bis 2, **dadurch gekennzeichnet, dass** man vor Erhöhung der Basizität einen nicht-basischen Hydrierkatalysator einsetzt.

4. Verfahren nach Anspruch 3, **dadurch gekennzeichnet, dass** der nicht-basische Katalysator auf nicht-basischen Trägermaterialien geträgert ist und/oder der Anteil an basischen Komponenten im nicht-basischen Hydrierkatalysator weniger als 0,5 Gew.-% bezogen auf die Gesamtmasse des Katalysators beträgt.

5. Verfahren nach mindestes einem der Anspruch 1 bis 4, **dadurch gekennzeichnet, dass** man die Basizität des Reaktionsgemisches erhöht, in dem man eine basische Verbindung als Lösung zufügt.

6. Verfahren nach Ansprüche 5, **dadurch gekennzeichnet, dass** man die Menge der zugefügten basischen Verbindung als Lösung so wählt, dass das Verhältnis der Masse der zugefügten basischen Verbindung zur Masse des 3-Cyano-3,5,5-trimethyl-cyclohexylimin im Eduktstrom 100 bis 10 000 zu 1 000 000 beträgt.

7. Verfahren nach mindestens einem der Ansprüche 1 bis 6, **dadurch gekennzeichnet, dass** man die Basizität des Reaktionsgemisches erhöht, in dem man als basische Verbindungen einen basischen Hydrierkatalysator einsetzt.

8. Verfahren nach Anspruch 7, **dadurch gekennzeichnet, dass** der Anteil von basischen Komponenten im basischen Hydrierkatalysator mindestens 0,5 Gew.-% bezogen auf die Gesamtmasse des basischen Hydrierkatalysators beträgt und/oder der Hydrierkatalysator auf einem basischen Träger geträgert wird.

9. Verfahren nach einem der Ansprüche 5 bis 7, wobei die basischen Komponenten im Hydrierkatalysator Oxide oder Hydroxide von Alkali- oder Erdalkalimetallen und/oder basische Trägermaterialien sind bzw. die basische Verbindung Oxide oder Hydroxide der Alkali- oder Erdalkalimetalle, Amine und/oder Ammoniumhydroxide darstellt.

10. Verfahren nach mindestens einem der Ansprüche 1 bis 9 **dadurch gekennzeichnet, dass** man einen kobalthaltigen Hydrierkatalysator verwendet.

11. Verfahren nach mindestens einem der Ansprüche 1 bis 10, **dadurch gekennzeichnet, dass** man die Umsetzung in zwei Stufen (Stufe I und Stufe II) durchführt.

12. Verfahren nach Anspruch 11, **dadurch gekennzeichnet, dass** man die Stufe I in einem Temperaturbereich von 50 bis 100°C bei einem Druck von 15 bis 300 bar durchführt und die Stufe II in einem Temperaturbereich von 70 bis 160°C bei einem Druck von 50 bis 300 bar durchführt.

13. Verfahren nach Anspruch 12, **dadurch gekennzeichnet, dass** man in Stufe I einen Ruthenium- und/oder Rhodium-haltigen Katalysator verwendet.

14. Verfahren nach mindestens einem der Ansprüche 11 bis 13, **dadurch gekennzeichnet, dass** man das Reaktionsgemisch mit der basischen Verbindung nach Stufe I in Kontakt bringt.

15. Verfahren nach mindestens einem der Ansprüche 11 bis 14, **dadurch gekennzeichnet, dass** man die Stufe I und/oder die Stufe II in zwei oder mehreren Teilstufen durchführt wird, wobei man das Reaktionsgemisch mit der basischen Verbindung frühestens nach der ersten Teilstufe der Stufe I in Kontakt bringt.

16. Verfahren nach mindestens einem der Ansprüche 11 bis 15, **dadurch gekennzeichnet, dass** man den Eduktstrom aufteilt, indem man einen Teil des Eduktstroms in die Stufe I und einen Teil des Eduktstroms direkt in die Stufe II leitet.

17. Verfahren nach mindestens einem der vorherigen Ansprüche, **dadurch gekennzeichnet, dass** man die Basizität erhöht, nachdem 5 bis 80% 3-Cyano-3,5,5-trimethyl-cyclohexylimins umgesetzt wurden.

## Claims

1. A continuous process for preparing 3-aminomethyl-3,5,5-trimethylcyclohexylamine by reacting a feed stream comprising 3-cyano-3,5,5-trimethylcyclohexylimine with hydrogen and ammonia over hydrogenation catalysts, wherein the basicity of the reaction mixture is increased during the reaction by bringing the reaction mixture into contact with a basic compound which is not ammonia and/or a basic catalyst after part of the 3-cyano-3,5,5-trimethylcyclohexylimine has been reacted.

2. The process according to claim 1, wherein the ratio of the mass of the basic compound to the mass of the 3-cyano-3,5,5-trimethylcyclohexylimine in the feed stream is less than 100 : 1 000 000 before the basicity is increased.

3. The process according to at least one of claims 1 and 2, wherein a nonbasic hydrogenation catalyst is used before increasing the basicity.

4. The process according to claim 3, wherein the nonbasic catalyst is supported on nonbasic support materials and/or the proportion of basic components in the nonbasic hydrogenation catalyst is less than 0.5% by weight, based on the total mass of the catalyst.

5. The process according to at least one of claims 1 to 4, wherein the basicity of the reaction mixture is increased by adding a basic compound as solution.

6. The process according to claim 5, wherein the amount of the basic compound added as solution is selected so that the ratio of the mass of the basic compound added to the mass of the 3-cyano-3,5,5-trimethylcyclohexylimine in the feed stream is 100-10 000 : 1 000 000.

7. The process according to at least one of claims 1 to 6, wherein the basicity of the reaction mixture is increased by using a basic hydrogenation catalyst as basic compound.

8. The process according to claim 7, wherein the proportion of basic components in the basic hydrogenation catalyst is at least 0.5% by weight based on the total mass of the basic hydrogenation catalyst and/or the hydrogenation catalyst is supported on a basic support.

9. The process according to any of claims 5 to 7, wherein the basic components in the hydrogenation catalyst are oxides or hydroxides of alkali or alkaline earth metals and/or basic support materials or the basic compound consists of oxides or hydroxides of the alkali or alkaline earth metals, amines and/or ammonium hydroxides.

10. The process according to at least one of claims 1 to 9, wherein a cobalt-comprising hydrogenation catalyst is used.

11. The process according to at least one of claims 1 to 10, wherein the reaction is carried out in two stages (stage I and stage II).

12. The process according to claim 11, wherein stage I is carried out in a temperature range from 50 to 100°C at a pressure of from 15 to 300 bar and stage II is carried out in a temperature range from 70 to 160°C at a pressure of from 50 to 300 bar.

13. The process according to claim 12, wherein a ruthenium- and/or rhodium-comprising catalyst is used in stage I.

14. The process according to at least one of claims 11 to 13, wherein the reaction mixture is brought into contact with the basic compound after stage I.

15. The process according to at least one of claims 11 to 14, wherein stage I and/or stage II are/is carried out in two or more substages, with the reaction mixture being brought into contact with the basic compound at the earliest after the first substage of stage I.

16. The process according to at least one of claims 11 to 15, wherein the feed stream is divided by introducing part of the feed stream into stage I and part of the feed stream directly into stage II.

17. The process according to at least one of the preceding claims, wherein the basicity is increased after from 5 to 80% of the 3-cyan-3,5,5-trimethyl-cyclohexylimine has been reacted.

## Revendications

1. Procédé continu pour la production de 3-aminométhyl-3,5,5-triméthylcyclohexylamine par mise en réaction d'un courant de produits de départ contenant de la 3-cyano-3,5,5-triméthyl-cyclohexylimine avec de l'hydrogène et de l'ammoniac sur des catalyseurs d'hydrogénation, **caractérisé en ce que** pendant la réaction on augmente la basicité du mélange réactionnel en mettant le mélange réactionnel en contact avec un composé basique différent de l'ammoniac et/ou un catalyseur basique, après qu'une partie de la 3-cyano-3,5,5-triméthyl-cyclohexylimine a réagi.

2. Procédé selon la revendication 1, **caractérisé en ce que** le rapport de la masse du composé basique à la masse de la 3-cyano-3,5,5-triméthyl-cyclohexylimine dans le courant de produits de départ, avant l'augmentation de la basicité, vaut de moins de 100 à 1 000 000.

3. Procédé selon au moins l'une quelconque des revendications 1 et 2, **caractérisé en ce qu'**avant l'augmentation de la basicité au utilise un catalyseur d'hydrogénation non basique.

4. Procédé selon la revendication 3, **caractérisé en ce que** le catalyseur non basique est fixé sur des matières de support non basiques et/ou la proportion de composants basiques dans le catalyseur d'hydrogénation non basique est inférieure à 0,5 % en poids, par rapport à la masse totale du catalyseur.

5. Procédé selon au moins l'une quelconque des revendications 1 à 4, **caractérisé en ce qu'**on augmente la basicité du mélange réactionnel en ajoutant un composé basique sous forme de solution.

6. Procédé selon la revendication 5, **caractérisé en ce qu'**on choisit la quantité du composé basique ajouté sous forme de solution de manière que le rapport de la masse du composé basique ajouté à la masse de la 3-cyano-3,5,5-triméthyl-cyclohexylimine dans le courant de produits de départ vaille de 100 à 10 000 : 1 000 000.

7. Procédé selon au moins l'une quelconque des revendications 1 à 6, **caractérisé en ce qu'**on augmente la basicité du mélange réactionnel en utilisant comme composés basiques un catalyseur d'hydrogénation basique.

8. Procédé selon la revendication 7, **caractérisé en ce que** la proportion de composants basiques dans le catalyseur d'hydrogénation basique est d'au moins 0,5 % en poids, par rapport à la masse totale du catalyseur d'hydrogénation basique et/ou le catalyseur d'hydrogénation est fixé sur un support basique.

9. Procédé selon l'une quelconque des revendications 5 à 7, dans lequel les composants basiques dans le catalyseur d'hydrogénation sont des oxydes ou hydroxydes de métaux alcalins ou alcalino-terreux et/ou des matières de support basiques ou bien le composé basique représente des oxydes ou hydroxydes des métaux alcalins ou alcalino-terreux, des amines et/ou des hydroxydes d'ammonium.

10. Procédé selon au moins l'une quelconque des revendications 1 à 9, **caractérisé en ce qu'**on utilise un catalyseur d'hydrogénation contenant du cobalt.

11. Procédé selon au moins l'une quelconque des revendications 1 à 10, **caractérisé en ce qu'**on effectue la réaction en deux étapes (étape I et étape II).

12. Procédé selon la revendication 11, **caractérisé en ce qu'**on effectue l'étape I dans une plage de température de 50 à 100 °C sous une pression de 15 à 300 bars et l'étape II dans une plage de température de 70 à 160 °C sous une pression de 50 à 300 bars.

13. Procédé selon la revendication 12, **caractérisé en ce que** dans l'étape I on utilise un catalyseur contenant du ruthénium et/ou du rhodium.

14. Procédé selon au moins l'une quelconque des revendications 11 à 13, **caractérisé en ce qu'**on met le mélange réactionnel en contact avec le composé basique après l'étape I.

15. Procédé selon au moins l'une quelconque des revendications 11 à 14, **caractérisé en ce qu'**on effectue l'étape I et/ou l'étape II en deux ou plus de deux étapes partielles, en mettant le mélange réactionnel en contact avec le composé basique au plus tôt après la première étape partielle de l'étape I.

16. Procédé selon au moins l'une quelconque des revendications 11 à 15, **caractérisé en ce qu'**on divise le courant de produits de départ en envoyant une partie du courant de produits de départ dans l'étape I et une partie du courant de produits de départ directement dans l'étape II.

17. Procédé selon au moins l'une quelconque des revendications précédentes, **caractérisé en ce qu'**on augmente la basicité après que 5 à 80 % de la 3-cyano-3,5,5-triméthyl-cyclohexylimine ont réagi.
